# EUROPEAN PATENT SPECIFICATION

(11) **EP 2 270 475 B1**
(45) Date of publication and mention of the grant of the patent: **28.03.2018**
(21) Application number: 09425365.5
(22) Date of filing: 22.09.2009
(51) Int. Cl.: G01N 21/64

(54) **Automated system for detecting aflatoxin in food**
Automatisiertes System zur Detektion von Aflatoxin in Nahrungsmitteln
Système automatisé de la détection d'aflatoxine dans l'aliment

(30) Priority: 30.06.2009 IT MI20091158
(43) Date of publication of application: 05.01.2011
(73) Proprietor: Star Ecotronics S.r.l., 20141 Milan (IT)
(72) Inventor: Ciminaghi, Alberto, 20141 Milan (IT)
(74) Representative: Fiorentino, Luca

(56) References cited:
- WO-A1-00/68670
- WO-A2-2006/123189
- US-A- 4 535 248
- US-A- 4 866 283

## Description

The present invention relates to an automated system and a method for detecting aflatoxin in food.

As it is known, the aflatoxins are mycotoxins, mainly produced by fungal stocks belonging to the *Aspergillus Flavus* species (only some stocks) and Aspergillus parasiticus (almost all the stocks) ubiquitarily diffused fungi, which can contaminate some food intended for human and animal feeding, among which, cereals (maize, wheat, soya bean, sorghum), oleaginous seeds, dried fruit and spices are the most affected.

The optimum conditions for the fungal mycelium growth are: a temperature ranging from 36 to 38°C, a substrate humidity at about 30% and ambient humidity at around 85%, whereas the largest production of toxins occurs at 24 to 30°C.

In particular, among the different kinds of known aflatoxins (B1, B2, G1, G2), the aflatoxin called B₁ is a powerful carcinogenic agent with a hepatic tropism.

As a result of the danger of such a substance, maximum limits of contamination by the total aflatoxins and AFB₁ (aflatoxin B₁), in particular, have been set, since it is detected mostly (up to a value of
Further, such methods imply a remarkably troublesome analysis and an extremely long preparation time of the samples to be analysed, besides a remarkable cost of the equipement used for the analysis itself.

As a result, such methods are usually employed for analysis, which do not involve real-time usable results.

An alternative method in respect to the above, based on the analysis of considerably heavier, and therefore more representative, samples makes use of an ultraviolet light.

More particularly, the principle on which the use of the UV radiation for estimating the aflatoxin contamination is based, consists of the property by the fungi, which belong to the *Aspergillus* genre to produce kojic acid (aflatoxin metabolite), which, when exposed to the UV light, emits a fluorescent radiation.

In the prior art analysis method for the fluorescent emissions, a sample of the product to be analysed - such as, for example, a specific number of cereal caryopsises - is placed on a sliding holder and irradiated by a UV lamp.

The luminescences, which occur on the caryopsis surface contaminated by aflatoxin, are detected by a camera, which sends the signal to a monitor.

An operator, when observing the above monitor, acts through a manual control (for example a switch) whenever it detects the presence of a luminescence, thereby recording the count of the number of fluorescent units in the sample under test.

Such a method implies a number of problems, related to the inaccuracy of the results : actually, the system is able to distinguish between samples with low or high levels of aflatoxin contamination, but it does not allow very accurate data to be obtained.

In more detail, said method measures only the number of output luminescences.

Moreover, in several cases, apparently fluorescent caryopsises were observed, also in absence of contamination.

Such a phenomenon is due to the fact that a possible presence of broken caryopsises, within the sample, causes the inner body thereof to reflect white light.

As it is known from the relevant literature, the fluorescent emission of kojic acid can take place only in the two light bands, green and red, therefore, if white light were output by the broken caryopsises, containing also the red and green bands, it results in detecting false positive by the test.

The analysis methods of the product through ultraviolet light of the prior art, therefore, actually overestimate the aflatoxin contamination level.

Therefore, is the object of the present invention to provide an alternative automated system and a method for detecting aflatoxin in food in respect to the conventional ones and, preferably, to solve the above said problems.

For example, a further object of the present invention is to realize an automated system for detecting aflatoxin in food, which is economically advantageous.

The present invention relates to an automated system for detecting aflatoxin in food and the relative method, as set forth in claims 1 and 8, to which reference is made for brevity.

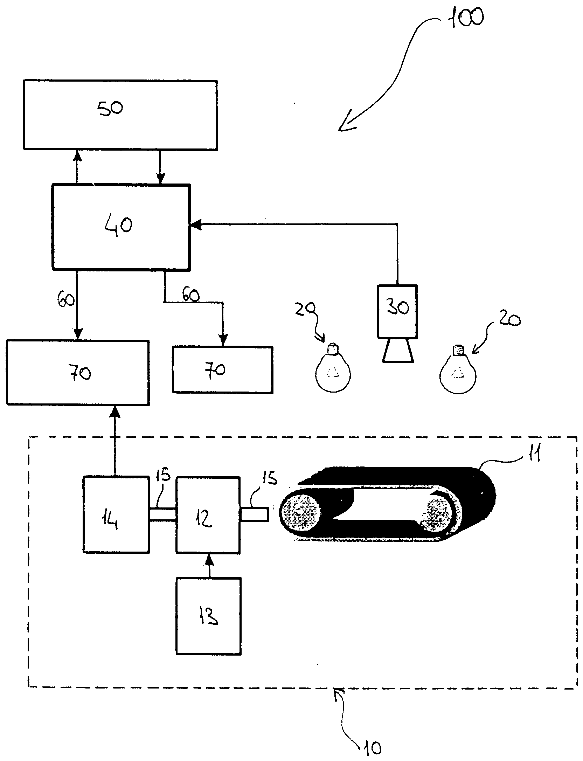

The invention is disclosed in detail here below, by way of example and not as a limitation, with reference to the appended figure 1, which shows a block diagram, which depicts a preferred embodiment of the detecting device of the present invention.

The automated system for detecting aflatoxins in fore, if white light were output by the broken caryopsises, containing also the red and green bands, it results in detecting false positive by the test.

The analysis methods of the product through ultraviolet light of the prior art, therefore, actually overestimate the aflatoxin contamination level.

Therefore, is the object of the present invention to provide an alternative automated system and a method for detecting aflatoxin in food in respect to the conventional ones and, preferably, to solve the above said problems.

For example, a further object of the present invention is to realize an automated system for detecting aflatoxin in food, which is economically advantageous.

The present invention relates to an automated system for detecting aflatoxin in food and the relative method, as set forth in claims 1 and 9, to which reference is made for brevity.

The invention is disclosed in detail here below, by way of example and not as a limitation, with reference to the appended figure 1, which shows a block diagram, which depicts a preferred embodiment of the detecting device of the present invention.

The automated system for detecting aflatoxins in food of the present invention is globally denoted by the reference numeral 100.

The automated system 100, as shown in figure 1, may comprise:
- a conveyor device 10;
- at least one source (20) of radiation for irradiating a food sample that is capable of generating corresponding fluorescent emissions;
- a device 30 for acquiring images;
- an encoder 14;
- a processing unit 40
- at least one monitor 50;
- at least a connection port 60 for at least an external peripheral 70.

In a preferred embodiment of the automated system of the present invention, said conveyor 10 comprises a conveyor belt 11, moved by an alternating current electric motor 12 through a shaft 15 and connected to an inverter 13, as well as said encoder 14.

Moreover, said source of radiation 20 is preferably formed by a Wood's lamp and the image acquisition device 30 comprises a high resolution digital camera. The above processing unit 40 can include an industrial PC 40, manages the system 100 automation for detecting aflatoxin, comprises, besides a main board, also a removable mass storage unit (CD or solid state) capable of storing data and images from 50 tests.

The processing unit 40 is equipped with an enablement module, adapt to enable the activation of the system, following an acknowledgement of an authorized user.

Still, in a preferred embodiment of the system 100 of the present invention, said monitor 50 can be composed by a LCD touch screen monitor, while said external peripheries 70 may comprise a printer for reproducing a report, related to data concerning the test carried out by the system.

The automated system 100 works, according to an example, as disclosed below.

The product to be analyzed (for example cereal caryopsises) is disposed on the conveyor belt 11, without any kind of pre-treatment.

The movement of the above belt 11 is preferably controlled by the encoder 14 fitted on the shaft 15 of the engine 12, which operates the belt 11 itself; said encoder 14 is, for example, set to send a pulse every tenth of a rotation degree of the shaft 15.

Such an expedient allows a high-resolution control of the conveyor belt 11 movement.

The high sensibility digital camera 30 is installed in the upper part of a tunnel - inside which the product under test travels on said conveyor belt 11 - and displays the passage of the product itself under the UV light output by the lamps 20. The camera 30 is run by the PC 40, which also governs the acquisition of images, one by one, counting the pulses of the above said encoder 14.

In the initial calibration step of the image acquisition system, the conveyor belt 11 is advanced step by step, and the system displays the image acquired by the camera 30 until said image is completely replaced by the following one. In this way, the number of pulses generated by the encoder 14 may be obtained, necessary so that the series of obtained pictures do not contain superimpositions or gaps between each other.

During the analysis step, therefore, the camera 30 acquires several images of corresponding portions of the product to be analyzed, while the conveyor belt 11 advances. It is to be observed that in each acquired image there may be caryopsises, which have fluorescence, besides other caryopsises which do not cause any fluorescence.

In particular, the images, acquired by the device 30, are digital images, which define a plurality of pixels, a level of fluorescence output being associated to each of them.

The processing unit 40 processes data and information (the level of fluorescence emission) associated to the pixels acquired by the camera 30. In particular, the processing unit 40 defines, based on the above said emission levels, and for each image or frame, a number of pixels per frame Npsi, to which an estimated presence of aflatoxin corresponds. This selection is carried out, for example, by comparing the various levels of fluorescence, measured after the acquisition of the images with a threshold level of fluorescence.

It is to be noted that the number of selected pixels per frame Npsi is representative of a portion of the surface of the i^{th} image estimated as contaminated. For example, the processing unit 40 computes, therefore, a total number of selected pixels Nps as the sum of the numbers of selected pixels related to all the frames. Preferably, in calculating the total number of selected pixels Nps, those values of the number of selected pixel per image Npsi lower than a predetermined threshold are excluded.

With respect to the step of selecting the pixels, which are considered as associated to the presence of contamination, a method is advantageously employed by the processing unit 40, which analyses separately the light fundamental emissions of the following bands: red R, green G and blue B. With regard to this, it is to be noted that the aflatoxin (or, better, its metabolite) emits fluorescence only at the wavelengths corresponding to the red chromatic component or to the green chromatic component.

An example of a possible separated analysis of the emissions can be the following: to each pixel a first light intensity related to the red chromatic component R, a second light intensity associated to the green chromatic component G and a third light intensity associated to the blue chromatic component B, are associated.

The processing unit 40 compares said light intensities to each other and selects pixels having at least one of said first and second associated light intensities, higher than the third light intensity, in order to obtain the above said selected pixels.

In more detail, (after the set parameters have been normalised) the luminosity of the blue chromatic component B is compared to that of the red chromatic component R, and if the blue chromatic component is higher, the red chromatic component R is removed from the algorithm. Subsequently, the luminosity of the blue chromatic component B is compared to that of the green chromatic component G. If the associated luminosity associated to the blue chromatic component B is higher than the green, the green chromatic component is removed from the algorithm. In such a situation, wherein the blue chromatic component B luminosity is predominant, the related pixel will be considered as not contaminated.

In cases where, as a result of the previous comparisons the luminance associated to the green G or red R is predominant and it exceeds a predetermined threshold, the related pixel will be selected as the pixel associated to the presence of contamination.

The algorithm described above prevents pixels with fluorescence not associated to wavelengths of green and red from being computed in the computing of the total selected pixels Nps and allows preventing false positives in tests to occur.

Subsequently, based on the total number of selected pixels Nps, the processing unit 40 provides information indicative of the quantity of aflatoxin (for example, expressed in ppb, parts per billion) present in the food sample under test.

In particular, in respect to assessing the quantity of aflatoxin present in the sample, the processing unit 40 comprises also a memory module, which stores digital data corresponding to a calibration curve adapt to correlate numerical values of total selected pixels Nps to values of quantity of aflatoxin. Such a calibration curve can be obtained through a calibration step, an example of which is reported below.

According to an example, the processing unit 40 can also govern the external peripheral 70 (for example a printer) in order to make a report available, which indicates, among the others: the number of images acquired, the total number of the contaminated caryopsises, the total number of selected pixels Nps concerned with the aflatoxin contamination and the estimated quantity of aflatoxin.

The automated system 100 for detecting aflatoxin of the present invention, has important advantages in respect to the systems used in the prior art.

It is to be noted that the method, which provides computing the number of pixels estimated as contaminants, allows an extremely accurate evaluation of the aflatoxin. In particular, the discrimination of the wavelengths, used for selecting the pixels, reduces or prevents false positives from occurring.

Moreover, the particular system disclosed is easy to use, as it does not require, among the others, skilled personnel. In addition, it is to be observed that the time for the analysis is extremely reduced and no special setting of the sample or the use of reagents are required.

A further advantage of the method employed by the system of the present invention is that, it consists of a non-destructive test, therefore one and the same sample can be possibly used for being analysed through further alternative methods.

Finally, a remarkable advantage related to the system 100 of the present invention is that said system is automated, hence it provides objective and reproducible results.

### Appendix: calibration method

For sake of completeness of the description, a calibration method of the system is disclosed below, based on the comparison between two tests: one using the HPLC method and one based on the emitted luminescence analysis following the exposition to UV light.

More in particular, a series of reference analysis was carried out (HPLC) for searching the quantity of aflatoxin B1 present in the sample.

The method employed was as follows:
- each sample, analysed by the HPLC method, was obtained making 10 logs on a 5 kg cereal sample;
- the 5 kg sample was processed horizontally 5 times by the automated system of the present invention, in order to obtain a "mean contamination value";
- from the above 5 kg sample, subsequently a 1 kg sample was extracted, which, in turn, was analysed 5 times by the automated system and it was defined as the representative sample, only in case the discovered mean fluorescence value (expressed in number of pixels) does not depart by a higher difference than 5% from the mean value obtained on the 5 kg;
- the 1 kg sample was then milled and properly homogenized, and two further samples of 50 gr. were obtained therefrom, which were analysed in a double HPLC. The mean value of the four readings was finally used for computing the calibration curve.

In respect to the HPLC analysis, 35 samples were tested, among which 16 to verify the calibration of the optical parameters of the automated system of the present invention. From the remaining 19 samples, a calibration curve was obtained, not forced at the axis origin.

A subsequent reduction of the samples of the series used to obtain the equation which discarded the samples having an excessive difference in respect to the trend curve, led to reduce the population to 11 points.

Finally, the calibration equation thereby obtained was included in the software of the automated system of the present invention.

From the foregoing, it is evident that the inventive concepts expressed are not limited to the illustrated application examples, but they can be advantageously adapted to other analogous applications.

The scope of the invention is not limited to the examples recited in the previous disclosure.

For example, it is possible to implement the method and employ the disclosed system also to identify other types of known aflatoxins, that is aflatoxins B2, G1 and G2 with design modification within the abilities of those skilled in the art and with a corresponding calibration of the equipment.

The present invention is, therefore, susceptible to a number of modifications and variants, all of which fall within in the inventive concept expressed in the appended claims, while the technical details can vary according to the needs.

## Claims

1. A system (100) for the detection of aflatoxin in food, the system comprising:
at least one source (20) of radiation for irradiating a food sample that is capable of generating corresponding fluorescent emissions, wherein said at least one source (20) comprises a Wood lamp for the emission of ultraviolet radiation;
a device (30) for the acquisition of images of said sample, wherein said acquisition device (30) is a digital camera;
wherein said images are digital images that define a first plurality of pixels, an emission level associated with the fluorescence being associated with each pixel,
**characterized in that** it comprises a processing unit (40) configured for defining, based on the emission levels, a number of selected pixels to which an estimated presence of aflatoxin corresponds, obtained by correlating the number of selected pixels to digital data corresponding to a calibration curve capable of correlating values of a selected number of pixels to quantity values of aflatoxin, and for supplying information indicating a quantity of aflatoxin present in the food sample based on said number of selected pixels, wherein a first luminous intensity related to the red chromatic component R, a second luminous intensity related to the green chromatic component G and a third luminous intensity related to the blue chromatic component B, are associated with each pixel; said processing unit being adapted to compare said luminous intensities to each other and to select pixels having associated at least one of the said first and second luminous intensities, higher than the third luminous intensity in such a way as to obtain said selected pixels.

2. A system (100) according to claim 1, wherein said processing unit (40) comprises a memory module that stores said digital data corresponding to said calibration curve capable of correlating values of selected numbers of pixels to quantity values of aflatoxin.

3. A system (100) according to at least one of the preceding claims, wherein:
- said aflatoxin is of the type included in the group consisting of: aflatoxin B2, G1, G2 and preferably B1;
- said food sample comprises a plurality of caryopses;
- said fluorescent emissions being associated with the presence of kojic acid in said caryopses;
- said processing unit (40) comprising an Industrial Personal Computer.

4. A system (100) according to at least one of the preceding claims, further comprising:
a conveyor (10) capable of moving the food sample;
an encoder (14) operatively associated with the conveyor for providing information indicating the positions taken by the food sample being moved;
wherein said processing unit (40), and the basis of on such information indicating the positions taken by the food sample, is capable of extracting from the data supplied by the acquisition device (30) nonoverlapping and adjacent images of a plurality of portions of the food sample.

5. A system (100) according to claim 4 when dependent on claim 3, wherein said one conveyor (10) is a conveyor belt on which the plurality of caryopses is distributed.

6. A system (100) according to at least one of the preceding claims, wherein said processing unit (40) is equipped with an enabling module capable of allowing the activation of the system following a recognition of an authorized user.

7. A system (100) according to at least one of the preceding claims, further comprising at least one peripheral device (70) connected to said processing unit (40) capable of making available to a user said information indicating the quantity of aflatoxin and setting parameters for said system and values measured on said food sample.

8. A process for the detection of aflatoxin comprising the steps of:
irradiating a food sample capable of generating corresponding fluorescent emissions through a Wood lamp for the emission of ultraviolet radiation;
acquiring digital images of the food sample that define a first plurality of pixels through a digital camera; a datum representing an emission level associated with the fluorescence being associated with each pixel;
processing the data representing emission levels to determine a number of selected pixels to which an estimated presence of aflatoxin corresponds obtained by correlating the number of selected pixels to digital data corresponding to a calibration curve capable of correlating values of a selected number of pixels to quantity values of aflatoxin; wherein a first luminous intensity related to the red chromatic component R, a second luminous intensity related to the green chromatic component G and a third luminous intensity related to the blue chromatic component B, are associated with each pixel; wherein said step of processing the data representing emission levels comprises comparing said luminous intensities to each other and for selecting pixels having associated at least one of the said first and second luminous intensities, higher than the third luminous intensity in such a way as to obtain said selected pixels;
processing the number of selected pixels and supplying information indicating a quantity of aflatoxin present in the food sample.

9. A process according to claim 8, further comprising a step of supplying the food sample in the form of a plurality of caryopses belonging to at least one of the following foods: cereals, corn, wheat, soya, sorghum, oleaginous seeds, dried fruit, spices.

## Patentansprüche

1. System (100) zum Erkennen von Aflatoxin in Lebensmittel, wobei das System Folgendes umfasst:
wenigstens eine Strahlungsquelle (20) zum Bestrahlen einer Lebensmittelprobe, die entsprechende fluoreszierende Emissionen erzeugen kann, wobei die wenigstens eine Quelle (20) eine Wood-Lampe zum Emittieren von ultravioletter Strahlung umfasst;
ein Gerät (30) zum Erfassen von Bildern der Probe, wobei das Erfassungsgerät (30) eine digitale Kamera ist;
wobei die Bilder digitale Bilder sind, die eine erste Mehrzahl von Pixeln definieren, wobei ein mit der Fluoreszenz assoziiertes Emissionsniveau mit jedem Pixel assoziiert ist,
**dadurch gekennzeichnet, dass** es eine Verarbeitungseinheit (40) umfasst, konfiguriert zum Definieren, auf der Basis der Emissionsniveaus, einer Anzahl von gewählten Pixeln, denen eine geschätzte Anwesenheit von Aflatoxin entspricht, erhalten durch Korrelieren der Anzahl von gewählten Pixeln mit digitalen Daten entsprechend einer Kalibrationskurve, die Werte einer gewählten Anzahl von Pixeln mit Mengenwerten von Aflatoxin korrelieren kann, und zum Zuführen von Informationen, die eine Menge an in der Lebensmittelprobe vorhandenem Aflatoxin auf der Basis der Anzahl von gewählten Pixeln anzeigen, wobei eine erste Leuchtstärke in Bezug auf die rote chromatische Komponente R, eine zweite Leuchtstärke in Bezug auf die grüne chromatische Komponente G und eine dritte Leuchtstärke in Bezug auf die blaue chromatische Komponente B mit jedem Pixel assoziiert sind; wobei die Verarbeitungseinheit so ausgelegt ist, dass sie die Leuchtstärken miteinander und mit gewählten Pixeln vergleicht, mit denen wenigstens eine der ersten und zweiten Leuchtstärken assoziiert ist, höher als die dritte Leuchtstärke, auf eine solche Weise, dass die gewählten Pixel erhalten werden.

2. System (100) nach Anspruch 1, wobei die Verarbeitungseinheit (40) ein Speichermodul umfasst, das die digitalen Daten speichert, die der Kalibrationskurve entsprechen, die Werte von gewählten Anzahlen von Pixeln mit Mengenwerten von Aflatoxin korrelieren kann.

3. System (100) nach wenigstens einem der vorherigen Ansprüche, wobei:
- das Aflatoxin von dem Typ ist, der in der Gruppe enthalten ist, die aus Aflatoxin B2, G1, G2 und vorzugsweise B1 besteht;
- die Lebensmittelprobe mehrere Karyopsen umfasst;
- die fluoreszierenden Emissionen mit der Anwesenheit von Kojisäure in den Karyopsen assoziiert sind;
- die Verarbeitungseinheit (40) einen Industrie-PC umfasst.

4. System (100) nach wenigstens einem der vorherigen Ansprüche, das ferner Folgendes umfasst:
einen Förderer (10), der die Lebensmittelprobe bewegen kann;
einen Encoder (14), der operativ mit dem Förderer assoziiert ist, um Informationen bereitzustellen, die die von der bewegten Lebensmittelprobe eingenommenen Positionen anzeigen;
wobei die Verarbeitungseinheit (40) und die Basis von auf solche Informationen, die die von der Lebensmittelprobe eingenommenen Positionen anzeigen, nicht überlappende und benachbarte Bilder aus einer Mehrzahl von Teilen der Lebensmittelprobe aus den von dem Erfassungsgerät (30) zugeführten Daten extrahieren kann.

5. System (100) nach Anspruch 4 in Abhängigkeit von Anspruch 3, wobei der eine Förderer (10) ein Förderband ist, auf dem mehrere Karyopsen verteilt sind.

6. System (100) nach wenigstens einem der vorherigen Ansprüche, wobei die Verarbeitungseinheit (40) mit einem Freigabemodul ausgestattet ist, das die Aktivierung des Systems nach einer Erkennung eines autorisierten Benutzers zulassen kann.

7. System (100) nach wenigstens einem der vorherigen Ansprüche, das ferner wenigstens ein Peripheriegerät (70) umfasst, das mit der Verarbeitungseinheit (40) verbunden ist, das einem Benutzer die Informationen zur Verfügung stellen kann, die die Menge an Aflatoxin und Einstellparameter für das System und an der Lebensmittelprobe gemessene Werte anzeigen.

8. Verfahren zum Erkennen von Aflatoxin, das die folgenden Schritte beinhaltet:
Bestrahlen einer Lebensmittelprobe, die entsprechende fluoreszierende Emissionen erzeugen kann, durch eine Wood-Lampe für die Emission von ultravioletten Strahlen;
Erfassen von digitalen Bildern der Lebensmittelprobe, die eine erste Mehrzahl von Pixeln definieren, durch eine digitale Kamera; wobei ein Referenzpunkt ein Emissionsniveau repräsentiert, das mit der mit jedem Pixel assoziierten Fluoreszenz assoziiert ist;
Verarbeiten der Emissionsniveaus repräsentierenden Daten zum Bestimmen einer Anzahl von gewählten Pixeln, denen eine geschätzte Anwesenheit von Aflatoxin entspricht, erhalten durch Korrelieren der Anzahl von gewählten Pixeln mit digitalen Daten entsprechend einer Kalibrationskurve, die Werte einer gewählten Anzahl von Pixeln mit Mengenwerten von Aflatoxin korrelieren kann; wobei eine erste Leuchtstärke in Bezug auf die rote chromatische Komponente R, eine zweite Leuchtstärke in Bezug auf die grüne chromatische Komponente G und eine dritte Leuchtstärke in Bezug auf die blaue chromatische Komponente B mit jedem Pixel assoziiert sind; wobei der Schritt des Verarbeitens der Emissionsniveaus repräsentierenden Daten das Vergleichen der Leuchtstärken miteinander und das Wählen von Pixeln beinhaltet, mit denen wenigstens eine der ersten und zweiten Leuchtstärken assoziiert ist, höher als die dritte Leuchtstärke, auf eine solche Weise, dass die gewählten Pixel erhalten werden;
Verarbeiten der Anzahl von gewählten Pixel und Zuführen von Informationen, die eine Menge an in der Lebensmittelprobe vorhandenem Aflatoxin anzeigen.

9. Verfahren nach Anspruch 8, das ferner einen Schritt des Zuführens der Lebensmittelprobe in Form von mehreren Karyopsen beinhaltet, die zu wenigstens einem der folgenden Lebensmittel gehören: Getreide, Mais, Weizen, Soja, Sorghum, ölhaltige Kerne, getrocknetes Obst, Gewürze.

## Revendications

1. Système (100) de détection d'aflatoxine dans les aliments, le système comprenant :
au moins une source (20) de rayonnement destinée à irradier un échantillon d'aliment qui est capable de générer des émissions fluorescentes correspondantes, ladite au moins une source (20) comprenant une lampe de Wood destinée à émettre un rayonnement ultraviolet ;
un dispositif (30) d'acquisition d'images dudit échantillon, ledit dispositif d'acquisition (30) étant une caméra numérique ;
lesdites images étant des images numériques qui définissent une première pluralité de pixels, un niveau d'émission associé à la fluorescence étant associé à chaque pixel,
**caractérisé en ce qu'**il comprend une unité de traitement (40) configurée pour définir, sur la base des niveaux d'émission, un nombre de pixels sélectionnés auxquels correspond une présence estimée d'aflatoxine, obtenue par corrélation du nombre de pixels sélectionnés avec des données numériques correspondant à une courbe d'étalonnage capable de mettre en corrélation des valeurs d'un nombre sélectionné de pixels avec des valeurs de quantité d'aflatoxine, et pour fournir des informations indiquant une quantité d'aflatoxine présente dans l'échantillon d'aliment sur la base dudit nombre de pixels sélectionnés, une première intensité lumineuse liée à la composante chromatique rouge R, une deuxième intensité lumineuse liée à la composante chromatique verte G et une troisième intensité lumineuse liée à la composante chromatique bleue B étant associées à chaque pixel ; ladite unité de traitement étant conçue pour comparer lesdites intensités lumineuses entre elles et pour sélectionner des pixels en ayant associé au moins l'une desdites première et deuxième intensités lumineuses, supérieure à la troisième intensité lumineuse, de manière à obtenir lesdits pixels sélectionnés.

2. Système (100) selon la revendication 1, ladite unité de traitement (40) comprenant un module de mémoire qui stocke lesdites données numériques correspondant à ladite courbe d'étalonnage capable de mettre en corrélation des valeurs de nombres sélectionnés de pixels avec des valeurs de quantité d'aflatoxine.

3. Système (100) selon au moins l'une des revendications précédentes :
- ladite aflatoxine étant du type inclus dans le groupe comprenant : l'aflatoxine B2, G1, G2 et de préférence B1 ;
- ledit échantillon d'aliment comprenant une pluralité de caryopses ;
- lesdites émissions fluorescentes étant associées à la présence d'acide kojique dans lesdits caryopses ;
- ladite unité de traitement (40) comprenant un ordinateur personnel industriel.

4. Système (100) selon au moins l'une des revendications précédentes, comprenant en outre :
un transporteur (10) capable de déplacer l'échantillon d'aliment ;
un encodeur (14) associé fonctionnellement au transporteur pour fournir des informations indiquant les positions prises par l'échantillon d'aliment qui est déplacé ;
ladite unité de traitement (40), et sur la base de ces informations indiquant les positions prises par l'échantillon d'aliment, étant capable d'extraire, des données fournies par le dispositif d'acquisition (30), des images adjacentes non chevauchantes d'une pluralité de parties de l'échantillon d'aliment.

5. Système (100) selon la revendication 4 lorsqu'elle dépend de la revendication 3, dans lequel ledit transporteur (10) est une bande transporteuse sur laquelle la pluralité de caryopses est distribuée.

6. Système (100) selon au moins l'une des revendications précédentes, dans laquelle ladite unité de traitement (40) est équipée d'un module d'activation capable de permettre l'activation du système à la suite de la reconnaissance d'un utilisateur autorisé.

7. Système (100) selon au moins l'une des revendications précédentes, comprenant en outre au moins un dispositif périphérique (70), relié à ladite unité de traitement (40), lequel est capable de mettre à la disposition d'un utilisateur lesdites informations indiquant la quantité d'aflatoxine et les paramètres de réglage dudit système et des valeurs mesurées sur ledit échantillon d'aliment.

8. Procédé de détection d'aflatoxine comprenant les étapes :
d'irradiation d'un échantillon d'aliment capable de générer des émissions fluorescentes correspondantes au moyen d'une lampe de Wood destinée à émettre un rayonnement ultraviolet ;
d'acquisition d'images numériques de l'échantillon d'aliment qui définissent une première pluralité de pixels au moyen d'une caméra numérique ; une donnée représentant un niveau d'émission associé à la fluorescence étant associée à chaque pixel ;
de traitement des données représentant les niveaux d'émission pour déterminer un nombre de pixels sélectionnés auxquels correspond une présence estimée d'aflatoxine obtenue par corrélation du nombre de pixels sélectionnés avec des données numériques correspondant à une courbe d'étalonnage capable de mettre en corrélation des valeurs d'un nombre sélectionné de pixels avec des valeurs de quantité d'aflatoxine ; une première intensité lumineuse liée à la composante chromatique rouge R, une deuxième intensité lumineuse liée à la composante chromatique verte G et une troisième intensité lumineuse liée à la composante chromatique bleue B étant associées à chaque pixel ; ladite étape de traitement des données, représentant les niveaux d'émission, comprenant la comparaison desdites intensités lumineuses entre elles et la sélection de pixels auxquels est associée au moins l'une desdites première et deuxième intensités lumineuses, supérieures à la troisième intensité lumineuse, de manière à obtenir lesdits pixels sélectionnés ;
de traitement du nombre de pixels sélectionnés et de fourniture d'informations indiquant une quantité d'aflatoxine présente dans l'échantillon d'aliment.

9. Procédé selon la revendication 8, comprenant en outre une étape de fourniture de l'échantillon d'aliment sous la forme d'une pluralité de caryopses appartenant à au moins l'un des aliments suivants : les céréales, le maïs, le blé, le soja, le sorgho, les graines oléagineuses, les fruits secs, les épices.
